# EUROPEAN PATENT SPECIFICATION

(11) **EP 1 496 352 B1**
(45) Date of publication and mention of the grant of the patent: **16.06.2010**
(21) Application number: 03077168.7
(22) Date of filing: 10.07.2003
(51) Int. Cl.: G01N 25/72, G01N 25/48, G01N 25/58, G01M 11/08, G01N 33/38

(54) **Method and apparatus for temperature monitoring of a physical structure**
Verfahren und Vorrichtung zur Temperaturüberwachung einer physikalischen Struktur
Procédé et dispositif pour surveiller la température d'une structure physique

(43) Date of publication of application: 12.01.2005
(73) Proprietor: Fortum OYJ, 02150 Espoo (FI)
(72) Inventor: Englund, Marja, 02760 Espoo (FI); Mitrunen, Asko, 02730 Espoo (FI)
(74) Representative: Stellberg, Hans Valter

(56) References cited:
- DE-A- 3 626 354
- FR-A- 2 559 262
- US-A- 5 041 987
- US-A1- 2002 018 510
- GROSSWIG S ET AL: "Distributed Fibre-Optic Temperature Sensing Technique (DTS) for Surveying Underground Gas Storage Facilities" OIL GAS EUROPEAN MAGAZINE, no. 4, 2001, pages 1-4, XP002264587
- ENGLUND MARJA ET AL: "DAM MONITORING USING A FIBRE-OPTIC TEMPERATURE SENSOR" INTERNATIONAL SEMINAR AND WORKSHOP SEINÄJOKI, 2 - 5 October 2000, pages 1-7, XP002264599
- RAO Y J ET AL: "Spatially-multiplexed fibre-optic Bragg grating strain and temperature sensor system based on interferometric wavelength-shift detection" ELECTRONICS LETTERS, IEE STEVENAGE, GB, vol. 31, no. 12, 8 June 1995 (1995-06-08), pages 1009-1010, XP006002927 ISSN: 0013-5194

## Description

### FIELD OF THE INVENTION

The invention relates to a method according to the preamble of claim 1.

The invention also relates to an arrangement according to the preamble of claim 14.

### BACKGROUND OF THE INVENTION

In large scale constructions quality assurance is highly important. For example, it is essential to verify that massive concrete structures do not break down or fall, while they are in use. Thus concrete structures should not contain cracks, spalls or honeycombing, which may weaken the structures.

During constructing cast concrete structures, high temperatures or large temperature differences within the concrete structure in the hardening phase may cause cracks or other defects in the structure. Thus, the quality of a concrete structure may be verified by means of temperature measurements during constructing the concrete structure. That is, temperature measurements during the construction can be used to control concreting, stripping, additional heating and protection in order to ensure in the best possible way, that the hardening phase goes as planned.

Thermoelement sensors are traditional means for measuring temperature of a concrete structure during the hardening phase. A more recent trend has been the use of optical fibre sensors in temperature measurements. Optical fibres are commonly used for data communications, but they can be employed also as temperature measurement sensors. Optical sensors have several advantages in comparison with electronic sensors, such as thermoelement sensors. They can be applied under adverse environmental conditions as well as at high temperatures. Thermoelement sensors are well suited for single-point measurements, but optical fibre sensors suit better for obtaining information about temperature differences and distribution within structures, since they effectively register temperatures over their whole length. For example a 50 m long optical fibre can be considered to correspond 50-200 thermoelement sensors. Especially in large and/or complicated massive structures the use of optical fibre sensors is thus advantageous.

Monitoring the temperature of a concrete structure during construction provides reasonable assurance of the quality of the concrete structure. However, defects can appear in the structures after hardening phase for example due to wearing, moisture, fluid leakages or environmental stress. Similar defects can appear in other physical structures, such as earth constructions.

Therefore, there is a need to monitor condition of structures also after their construction has been completed in order to identify defects appearing in later phases. To some extent temperature measurements can be used also for this purpose.

For example, document EP 0 572 238 B1 describes a method of monitoring temperature and condition of a blast furnace by means of optical fibre sensors and document EP 0 555 846 A2 describes a structure for detecting temperature abnormality in a fluid pipe.

Document US 2002/0018510 A1 presents the use of time-resolved infrared radiometry as an inspection method for composite structures. The use of a fibre-optic temperature sensing technique for inspecting gas storage facilities is discussed in S. Grosswig et al.: *Distributed Fibre-optic Temperature Sensing Technique (DTS) for Surveying Underground Gas Storage Facilities,* Oil Gas European Magazine 4/2001. Another fiber-optic temperature sensor system is discussed in

Y.J. Rao et al.: Spatially-multiplexed fibre-optic Bragg grating strain and temperature sensor system based on interferometric wavelength-shift detection, Electronics Letters, 8th June 1995, Vol. 31, No. 12, p. 1009. And, the use of a fibre-optic sensor for dam monitoring has been discussed in the article originating from the inventor of the present application, namely Marja Englund et al.: Dam monitoring using a fibre-optic temperature sensor, International Seminar and Workshop, Seinäjoki, Finland, October 2-5, 2000.

However, there are many defects that cannot be identified by means of temperature measurements. For example, if there is a fluid leakage and the temperature of the fluid is equal to the temperature of the structure, temperature monitoring does not give any indication of the leakage.

A method of detecting fluid leaks in a dumpsite, using a meandering cable that comprises an optical fibre temperature sensor and a thermal cable, is described in EP 0672903.

Moreover, there is a need to provide a method for detecting defects in grouting concrete and/or for monitoring grouting quality. Grouting is a method of constructing concrete, wherein prestressing steel is placed into a duct within a concrete structure and concrete is grouted into the duct to fill the space between the prestressing steel and the duct. The space between the prestressing steel and the duct should be fully filled, but sometimes there are holes or coarse areas or a so called rock pocket in there, which may weaken the concrete structure. It is difficult to detect these holes or coarse areas by means of prior art methods and therefore a new method is needed.

### SUMMARY OF THE INVENTION

An object of the invention is to improve monitoring the condition of a substantially homogenous physical structure, such as an earth construction, an earth filled dam, structures of a district heating network, or a building structure, such as a structure made of concrete or conductive concrete.

The invention is based on using variation of heat distribution in a physical structure for detecting defects and/or non-homogeneity in the structure. For example thermal conductivities of wet and dry structures are different and thermal conductivities of a structure with cracks or spalls and an unbroken structure are different.

The characterics of the inventive method are disclosed in the characterising portion of claim 1.

The characteristics of the inventive arrangement are disclosed in the characterising portion of claim 14.

Dependent claims contain preferable embodiments of the invention. The subject matter contained in dependent claims relating to a particular aspect of the invention is also applicable to other aspects of the invention.

Advantages of an embodiment of the invention are that it provides an improved method for monitoring physical structures and that structures can be monitored over their whole lifecycle. Also the condition of structures, which are already in use, can be analysed. In principle, monitoring can be continuous, that is, measurements are repeated periodically, or the measurements can be done only when they are considered necessary. Moreover, providing a timely and relatively accurate estimate of damages in physical structures is enabled.

In prior art solutions, temperature measurements may have been used for identifying defects in a structure. In case defects have been detected, the temperature of the structure may have been changed in order to minimize the defect. Whereas, the invention provides a method, wherein changing the temperature of the structure is part of the method of monitoring the condition of the structure. Nevertheless, the method of the invention does not prevent using an additional change of temperature for minimizing defects detected in the structure.

### BRIEF DESCRIPTION OF THE DRAWINGS

Embodiments of the invention will now be described by way of example with reference to the accompanying drawings in which:
- Figure 1: is a flow diagram illustrating a method according to an embodiment of the invention;
- Figure 2: illustrates an arrangement according to an embodiment of the invention;
- Figures 3A-3C: illustrate by way of example certain preferred ways for assembling a thermal cable in proximity of an optical fibre sensor; and
- Figure 4: illustrates by way of example a simplified block diagram of a device that can be used in the invention.

### DETAILED DESCRIPTION

In order to clarify the technology related to the invention, optical fibres and the use of optical fibres as temperature sensors are first briefly discussed. Optical fibre comprises a core and a cladding made of glass having different refraction indexes. The function of an optical fibre is based on the refraction and reflection of light in the interface of the core and the cladding.

When an optical fibre is used for data communications, which is the most common application field of optical fibres, the fibre and cabling of the fibre are selected so that environment affects transferred data as little as possible. Whereas when an optical fibre is used as a sensor, the selection of materials is such that the variable to be measured has a controlled effect on the light travelling in the fibre. Then, changes in light intensity, phase, wavelength or polarisation can be used for determining the value of the variable to be measured.

A temperature measurement by means of an optical fibre sensor employs for example analysing Raman backscattered signals caused when a laser pulse is travelling in the optical fibre. A short laser pulse is sent into the optical fibre from one end of the fibre. As a result of spontaneous Raman scattering, anti-Stokes and Stokes photons are generated along the fibre. A fraction of these scattered photons propagate back and are detected by a photodetector. Especially the generation of anti-Stokes photons is sensitive to the temperature of the environment. Therefore, by means of analysing the backscattered Stokes and anti-Stokes photons, the temperature along the fibre can be calculated. The point of the optical fibre in which a certain temperature value was measured can be identified on the basis of the speed of light and the time between sending the light pulse and detecting the backscattered photons. In order to obtain more than one sample for the measurement points, the light pulse is sent repeatedly and the repetition frequency is selected so that backscattered signals originated from different light pulses do not mix up.

The accuracy of temperature measurement results along the length of the optical fibre depends on the characteristics of the fibre, but in any case an optical fibre sensor can be considered to be a full-length sensor.

It must be noted that the above method of measuring temperature by means of an optical fibre sensor is presented as an example only. The exact details of the temperature measurement arrangement are not relevant, but any suitable temperature measurement implementation, which provides distributed temperature measurement, can be used in connection with the invention. The temperature measurement can be based on any other suitable physical phenomenon, too. For example Brillouin-based fibre optic distributed temperature sensors can be used.

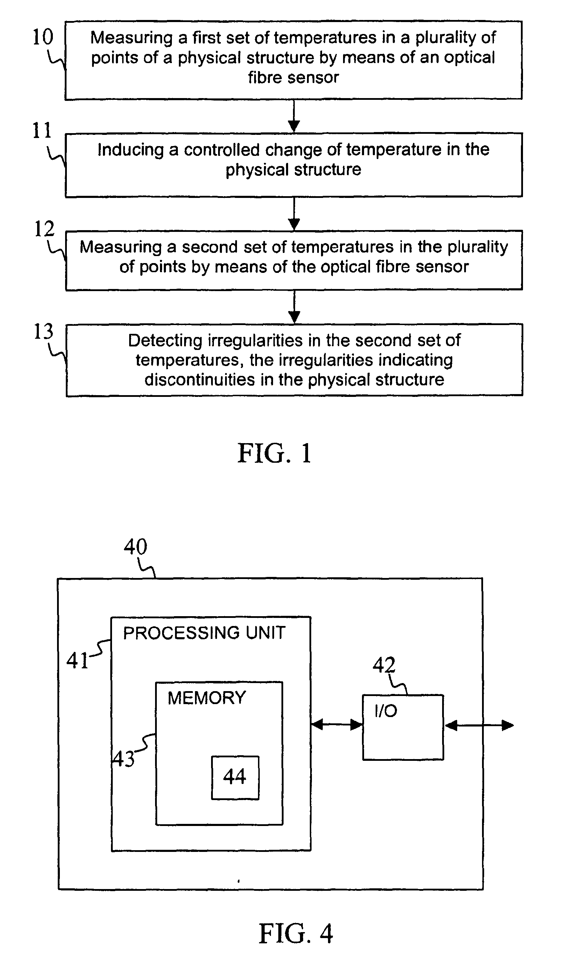

Figure 1 is a flow diagram illustrating a method according to the invention for monitoring the condition of a substantially homogenous physical structure. It is assumed that an optical fibre temperature sensor is cast or buried into the physical structure or fitted on the surface of it in order to measure temperature of the physical structure. Preferably, the optical fibre is assembled so that temperature is measured over the full length of the structure or over a desired two- or three-dimensional plane of the structure.

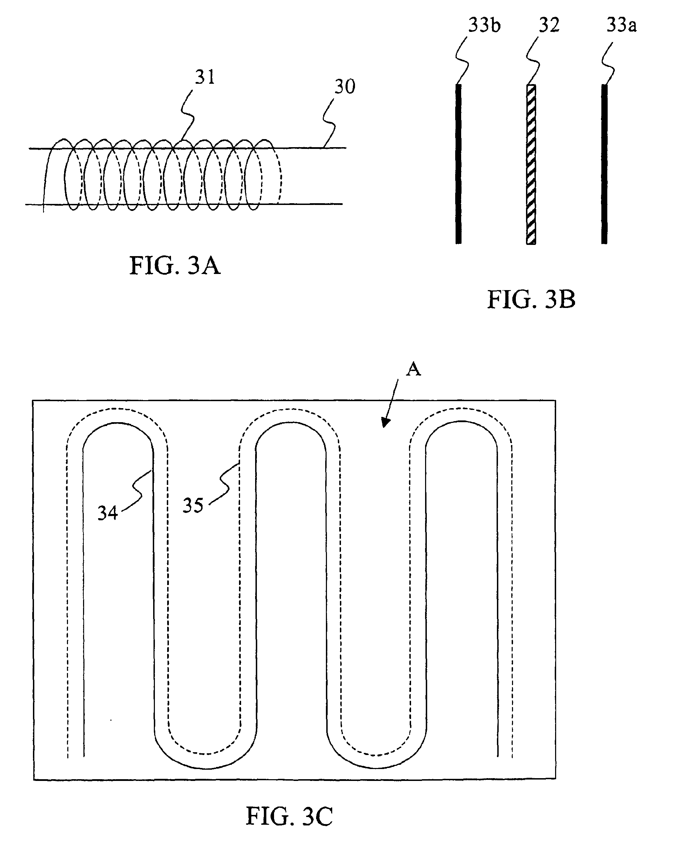

In step 10, a first set of temperatures is measured in a plurality of points of the physical structure by means of the optical fibre sensor. As an optical fibre sensor can be considered to be a full-length sensor, the points in which temperature is measured may be in fact next to each other. Then in step 11, a controlled change of temperature is caused in said physical structure. Preferably the physical structure is heated, but equally it can be cooled down. The change of temperature can be caused for example by means of a thermal cable assembled in proximity of the optical fibre sensor. The use of thermal cables is further discussed below in connection with Figure 3. Alternatively, if the physical structure is conductive (for example conductive concrete), heat can be conveyed into the structure by means of its conductivity. In that case it is enough to attach heating electrodes on the surface of the physical structure and the conductivity of the structure takes care of distributing the heat inside the structure. Still other possibility is to use hot- or cold-liquid pipeline inside the structure for causing the change of temperature.

In step 12, a second set of temperatures are measured in the plurality of points of the physical structure by means of the optical fibre sensor and in step 13 the second set of temperatures is analysed and irregularities in the temperatures are detected, the irregularities indicating at least one discontinuity in the physical structure. As the physical structure to be monitored is substantially homogenous in normal circumstances, heat should be somewhat evenly distributed in the structure. If heat is not distributed evenly, it can be assumed that there is some defect or discontinuity in the structure. Such discontinuity or defect may be for example a hole, a coarse area, a rock pocket, entrapped air, a crack, a spall, honeycombing, a frozen area, moisture, a leakage, a bend, or a dislocation in the physical structure, a foreign object within the structure, or running fluid inside or on the surface of the physical structure. Therefore, according to certain embodiments of the invention, the method of monitoring the condition of a substantially homogenous physical structure is used for detecting and/or locating honeycombing in a concrete structure and/or for analysing grouting quality in a concrete structure.

If the change of temperature caused in step 11 is even over the physical structure, discontinuities in the structure can be detected by calculating difference in the temperature of in various points of the structure before and after changing the temperature and by detecting variation in the temperature difference between different points. That is, a difference between the first and second sets of measured temperatures is calculated. A relatively large or small temperature difference in some point indicates non-homogeneity of the structure or a defect in the structure in that point. In order to ensure reliable results, it is preferred that only discontinuities, which are indicated by more than one measurement point or which are indicated by relatively large deviation from normal results, are taken into account. In this way normal, minor variation in the material, of which the structure is made, does not cause errors in the monitoring results.

It must be noted that magnitude of the controlled change of temperature may be different in different parts of the physical structure. However, in that case the differences in the change of temperature need to be taken into account in analysing measured temperatures.

Moreover, heating the structure and temperature measurements can be repeated and/or they can both be continuous and/or the heating magnitude may be changed. This way, for example, changes in the structure over a certain time period can be identified. For example, if there is moisture in the structure, heating should evaporate the moisture. Thus a continuous leak in the structure can be distinguished from occasional moisture by detecting, if the heating dries the structure. Different heating magnitudes and different lengths of the heating time can be used for analysing the extent of a leakage in the structure. Also the temperature of the environment can be taken into account in analysing different temperatures of the structure.

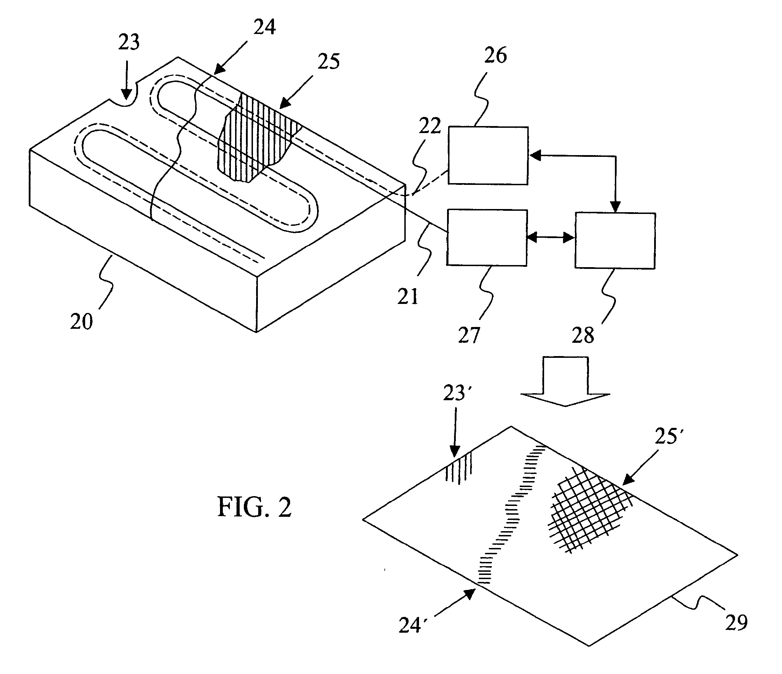

Figure 2 illustrates an arrangement according to an embodiment of the invention. Therein, the condition of a concrete structure 20 is monitored. The Figure 2 shows a cross sectional view of the concrete structure showing an optical fibre sensor 21, which has been assembled into the concrete structure. The optical fibre sensor is bent to cover a cross sectional plane of the concrete structure so that a temperature versus a location chart over the cross sectional plane can be obtained by means of the optical fibre sensor. The optical fibre sensor 21 is coupled to a temperature measurement unit 27, which is adapted to send a light pulse into the optical fibre sensor and to detect and analyse backscattered light from the sensor for determining temperature in different points of the concrete structure. The temperature measurement unit 27 preferably comprises a source of light and a detector (not shown), which are functionally coupled to the optical fibre, and a signal processor (not shown) for providing the temperature measurement results.

A thermal cable 22 is assembled next to the optical fibre sensor within the concrete structure 20. The thermal cable is coupled to a temperature-controlling unit 26, which is adapted to send a heat pulse into the thermal cable in order to cause a change of temperature in the concrete.

The temperature measurement unit 27 and the temperature-controlling unit 26 are both functionally coupled to a computing device 28, which comprises analysing means for analysing temperature measurement results obtained from the temperature measurement unit 27. These analysing means are preferably implemented as a suitable combination of hardware and software components; for example as a processing unit and a computer program code executable in the processing unit.

The analysing means produce and output a two-dimensional chart 29 over the cross sectional plane of the differences in the temperatures before and after heating the concrete structure. The areas 23', 24' and 25', where the differences in the temperatures are relatively large or small are shaded in the chart 29. The shading 23' indicates a spall 23 in the concrete, the shading 24' indicates a crack 24 and the shading 25' indicates a wet area 25.

Preferably the computing device 28 also controls the temperature measurement unit 27 and the temperature-controlling unit 26 in order to set the timing of temperature measurements and heating as desired.

If the concrete structure in Figure 2 is replaced with a structure made of conductive concrete (or some other conductive material), the thermal cable 22 can be replaced with heating electrodes, which are attached to the surface of the structure. Conductivity of the structure then conveys the heat within the structure and differences in heat distribution indicating defects in the structure can be detected by measuring the temperature by means of the optical fibre sensor as described above.

As discussed above, the controlled change of temperature in the physical structure may be caused for example by means of a thermal cable, which is beforehand assembled substantially in proximity of the optical fibre sensor. Figures 3A-3C illustrate by way of example certain preferred ways for assembling a thermal cable in proximity of an optical fibre sensor. In Figure 3A a thermal cable 31 has been wound around an optical fibre sensor 30, and in Figure 3B two parallel thermal cables 33a and 33b have been fitted next to an optical fibre sensor 32 onto opposite sides of the sensor. Whereas, in Figure 3C an optical fibre sensor 34 has been bent to effectively cover an area A in a two-dimensional plane and a thermal cable 35 has been bent into the same form with the optical fibre sensor and fitted next to the sensor. Any one of these assemblies can be cast or buried into the physical structure or fitted on the surface of the physical structure; the former concerning especially assemblies during the construction of the structure and the latter concerning mainly assemblies after the construction of the structure. Clearly, a thermal cable can be assembled in any other suitable way, too.

Figure 4 illustrates by way of example a simplified block diagram of a device 40 that can be used in the invention, which device 40 may be for example a general-purpose computer or some other suitable computing device. According to an embodiment of the invention the device 40 comprises a processing unit 41 comprising memory 43. The processing unit is functionally coupled to an I/O interface 42, through which the device 40 communicates with other devices.

A computer program 44 to be executed in the processing unit 41 is stored in the memory 43. The computer program 44 comprises program code for controlling temperature measurement means comprising an optical fibre sensor and controlling means for causing a change of temperature, such as the temperature measurement unit 26 and the temperature-controlling unit 27 shown in Figure 2. The computer program 44 controls the temperature measurement means to obtain a set of first measured temperature values for a plurality of points in a physical structure, the temperature-controlling means to cause a controlled change of temperature in the physical structure, and again the temperature measurement means to obtain a set of second measured temperature values for a plurality of points in the physical structure after the controlled change of temperature has been induced into the physical structure. Furthermore, the computer program comprises program code for detecting irregularities in the second measured values, while the irregularities indicate discontinuities, such as holes, coarse areas, moisture or some other defect, in said physical structure.

The computer program may comprise also program code for outputting a graphical representation indicating the discontinuities detected in the physical structure. Further, the computer program may calculate, for the plurality of points in which the temperature is measured, difference between the sets of first and second measured temperature values. Variation in this difference between the plurality of points then indicates the irregularities and thereby the discontinuities in the physical structure.

Particular implementations and embodiments of the invention have been described above by using monitoring the condition of concrete structures as an illustrative example. It is clear to a person skilled in the art that the invention is not restricted to concrete structures only or to details of the embodiments presented above, but that it can be used for monitoring any other suitable physical structure, which is substantially homogenous in normal conditions, and implemented in other embodiments using equivalent means without deviating from the characteristics of the invention. The scope of the invention is only restricted by the attached patent claims.

## Claims

1. A method for monitoring the condition of a substantially homogenous physical structure (20), said method comprising the steps of:
- measuring (10) a temperature of said physical structure by means of an optical fibre sensor (21, 30, 32, 34) for obtaining a set of measured temperatures in a plurality of points of said physical structure,
- causing (11) a controlled change of temperature in said physical structure by means of a thermal cable (22, 31, 33a, 33b, 35) assembled in proximity of said optical fibre (21, 30, 32, 34), said controlled change of temperature being even over said physical structure,
- detecting (13) irregularities in said measured temperatures, said irregularities indicating at least one discontinuity in said physical structure, said detecting of said irregularities **characterised by** the steps of:
- obtaining for said plurality of points a first set of measured temperatures (10) before said controlled change of temperature and a second set of measured temperatures (12) after said controlled change of temperature,
- calculating, for said plurality of points, a difference between said first and second sets of measured temperatures, and
- detecting variation in said difference between said plurality of points, said variation indicating said at least one discontinuity in said physical structure.

2. A method according to claim 1, wherein the magnitude of said controlled change of temperature varies over said physical structure and said variation of the magnitude is taken into account in detecting said irregularities.

3. A method according to claim 1 or 2, wherein said physical structure is made of concrete or conductive concrete.

4. A method according to claim 3, wherein said method is used for detecting and/or locating honeycombing in the concrete or conductive concrete and/or for analysing grouting quality in the concrete or conductive concrete.

5. A method according to claim 1 or 2, wherein said physical structure is a structure of a district heating network.

6. A method according to claim 1 or 2, wherein said physical structure is made of conductive concrete and said controlled change of temperature is caused by means of conductive parts in the concrete.

7. A method according to claim 1 or 2, wherein said physical structure is an earth construction.

8. A method according to claim 1 or 2, wherein said physical structure is an earth filled dam.

9. A method according to any one of claims 1 to 8, wherein said discontinuity is one of the following: a hole, a coarse area, a rock pocket, entrapped air, a crack, a spall, honeycombing, a frozen area, moisture, a leakage, a bend, or a dislocation in the physical structure, a foreign object within the structure, or running fluid inside or on the surface of the physical structure.

10. A method according to any one of claims 1 to 9, wherein said method is used for monitoring the condition of the physical structure after construction of said physical structure has been finished.

11. A method according to any one of claims 1 to 10, wherein said method is used for monitoring the condition of the physical structure while said physical structure is in normal use.

12. A method according to any one of claims 1 to 11, wherein magnitude of said controlled change of temperature is pre-determined.

13. A method according to any one of claims 1 to 12, wherein magnitude of said controlled change of temperature is independent of the temperature measurement results.

14. An arrangement for monitoring the condition of a substantially homogenous physical structure (20), comprising:
- temperature measurement means (27) comprising an optical fibre sensor (21, 30, 32, 34) for measuring temperature of said physical structure for obtaining a set of measured temperatures in a plurality of points of said physical structure,
- a thermal cable (22, 31, 33a, 33b, 35) assembled in proximity of said optical fibre (21, 30, 32, 34) for causing a controlled change of temperature in said physical structure,
- said thermal cable (22, 31, 33a, 33b, 35) being coupled to a temperature-controlling unit (26), which is adapted to send a heat pulse into the thermal cable (22, 31, 33a, 33b, 35) in order to cause a change of temperature, whose magnitude is even over said physical structure,
- means (28) for detecting irregularities in said measured temperatures, said irregularities indicating at least one discontinuity in said physical structure,
- said means (28) for detecting said irregularities **characterised by** being adapted to:
- obtain, for said plurality of points, a first set of measured temperatures before said controlled change of temperature and a second set of measured temperatures after said controlled change of temperature,
- calculate, for said plurality of points, difference between said first and second sets of measured temperatures, and
- detect variation in said difference between said plurality of points, said variation indicating said at least one discontinuity in said physical structure.

## Patentansprüche

1. Verfahren zum Überwachen des Zustands einer im Wesentlichen homogenen physikalischen Struktur (20), wobei das Verfahren die Schritte aufweist:
- Messen (10) einer Temperatur der physikalischen Struktur mit Hilfe eines Lichtleitersensors (21, 30, 32, 34), um einen Satz von gemessenen Temperaturen an einer Vielzahl von Punkten der physikalischen Struktur zu erhalten,
- Verursachen (11) einer gesteuerten Temperaturänderung in der physikalischen Struktur mit Hilfe eines Heizdrahts (22, 31, 33a, 33b, 35), der in der Nähe des Lichtleiters (21, 30, 32, 34) angebracht ist, wobei die gesteuerte Temperaturänderung gleichmäßig über die physikalische Struktur ist,
- Erfassen (13) von Unregelmäßigkeiten in den gemessenen Temperaturen, wobei die Unregelmäßigkeiten zumindest eine Diskontinuität in der physikalischen Struktur anzeigen, und wobei das Erfassen der Unregelmäßigkeiten durch die Schritte **gekennzeichnet** ist:
- Erhalten eines ersten Satzes von gemessenen Temperaturen (10) vor der gesteuerten Temperaturänderung und eines zweiten Satzes von gemessenen Temperaturen (12) nach der gesteuerten Temperaturänderung für die Vielzahl von Punkten,
- Berechnen einer Differenz zwischen dem ersten und zweiten Satz von gemessenen Temperaturen für die Vielzahl von Punkten, und
- Erfassen einer Variation in der Differenz zwischen der Vielzahl von Punkten, wobei die Variation zumindest eine Diskontinuität in der physikalischen Struktur anzeigt.

2. Verfahren nach Anspruch 1, wobei die Größe der gesteuerten Temperaturänderung über die physikalische Struktur variiert, und die Variation der Größe bei dem Erfassen der Irregularitäten berücksichtigt wird.

3. Verfahren nach Anspruch 1 oder 2, wobei die physikalische Struktur aus Beton oder leitfähigem Beton gemacht ist.

4. Verfahren nach Anspruch 3, wobei das Verfahren zum Erfassen und/oder Lokalisieren von Rissen in dem Beton oder leitfähigen Beton und/oder zum Analysieren einer Zementierqualität in dem Beton oder leitfähigen Beton verwendet wird.

5. Verfahren nach Anspruch 1 oder 2, wobei die physikalische Struktur eine Struktur eines Fernwärmenetzes ist.

6. Verfahren nach Anspruch 1 oder 2, wobei die physikalische Struktur aus leitfähigem Beton gemacht ist, und die gesteuerte Temperaturänderung mit Hilfe leitfähiger Teile in dem Beton verursacht wird.

7. Verfahren nach Anspruch 1 oder 2, wobei die physikalische Struktur eine Erdkonstruktion ist.

8. Verfahren nach Anspruch 1 oder 2, wobei die physikalische Struktur ein mit Erde gefüllter Damm ist.

9. Verfahren nach einem der Ansprüche 1 bis 8, wobei die Diskontinuität eine der Folgenden ist: ein Loch, ein grober Bereich, ein Kiesnest, eingeschlossene Luft, ein Bruch, ein Bruchstein, ein Riss, ein gefrorener Bereich, Feuchtigkeit, ein Leck, eine Krümmung, oder eine Versetzung in der physikalischen Struktur, ein Fremdobjekt in der Struktur, oder laufende Flüssigkeit innerhalb oder auf der Oberfläche der physikalischen Struktur.

10. Verfahren nach einem der Ansprüche 1 bis 9, wobei das Verfahren verwendet wird, um den Zustand der physikalischen Struktur zu überwachen, nachdem eine Konstruktion der physikalischen Struktur beendet wurde.

11. Verfahren nach einem der Ansprüche 1 bis 10, wobei das Verfahren verwendet wird, um den Zustand der physikalischen Struktur zu überwachen, während die physikalische Struktur in normalem Gebrauch ist.

12. Verfahren nach einem der Ansprüche 1 bis 11, wobei eine Größe der gesteuerten Temperaturänderung vorbestimmt ist.

13. Verfahren nach einem der Ansprüche 1 bis 12, wobei eine Größe der gesteuerten Temperaturänderung unabhängig von den Temperaturmessergebnissen ist.

14. Eine Anordnung zum Überwachen des Zustands einer im Wesentlichen homogenen physikalischen Struktur (20), mit:
- einer Temperaturmesseinrichtung (27) mit einem Lichtleitersensor (21, 30, 32, 34) zum Messen einer Temperatur der physikalischen Struktur, um einen Satz von gemessenen Temperaturen an einer Vielzahl von Punkten der physikalischen Struktur zu erhalten,
- einem Heizdraht (22, 31, 33a, 33b, 35), der in der Nähe des Lichtleiters (21, 30, 32, 34) angebracht ist, um eine gesteuerte Temperaturänderung in der physikalischen Struktur zu verursachen,
- wobei der Heizdraht (22, 31, 33a, 33b, 35) mit einer Temperatursteuereinheit (26) verbunden ist, die angepasst ist, einen Heizpuls in den Heizdraht (22, 31, 33a, 33b, 35) zu senden, um eine Temperaturänderung zu verursachen, deren Größe über die physikalische Struktur gleichmäßig ist,
- einer Einrichtung (28) zum Erfassen von Irregularitäten in den gemessenen Temperaturen, wobei die Irregularitäten zumindest eine Diskontinuität in der physikalischen Struktur anzeigen,
- wobei die Einrichtung (28) zum Erfassen der Irregularitäten **dadurch gekennzeichnet ist, dass** sie angepasst ist:
- für die Vielzahl von Punkten einen ersten Satz von gemessenen Temperaturen vor der gesteuerten Temperaturänderung und einen zweiten Satz von gemessenen Temperaturen nach der gesteuerten Temperaturänderung zu erfassen,
- für die Vielzahl von Punkten eine Differenz zwischen dem ersten und zweiten Satz von gemessenen Temperaturen zu berechnen, und
- eine Variation in der Differenz zwischen der Vielzahl von Punkten zu erfassen, wobei die Variation die zumindest eine Diskontinuität in der physikalischen Struktur anzeigt.

## Revendications

1. Procédé de surveillance de l'état d'une structure physique sensiblement homogène (20), ledit procédé comprenant les étapes suivantes :
- la mesure (10) d'une température de ladite structure physique au moyen d'un capteur à fibres optiques (21, 30, 32, 34) pour obtenir un ensemble de températures mesurées en une pluralité de points de ladite structure physique,
- le changement contrôlé (11) de la température dans ladite structure physique au moyen d'un câble thermique (22, 31, 33a, 33b, 35) assemblé à proximité de ladite fibre optique (21, 30, 32, 34), ledit changement contrôlé de température étant uniforme sur ladite structure physique,
- la détection (13) d'irrégularités dans lesdites températures mesurées, lesdites irrégularités indiquant au moins une discontinuité dans ladite structure physique, ladite détection desdites irrégularités étant **caractérisée par** les étapes suivantes :
- l'obtention pour ladite pluralité de points d'un premier ensemble de températures mesurées (10) avant ledit changement contrôlé de température et d'un second ensemble de températures mesurées (12) après ledit changement contrôlé de température,
- le calcul, pour ladite pluralité de points, d'une différence entre ledit premier ensemble de températures mesurées et ledit second ensemble de températures mesurées, et
- la détection d'une variation dans ladite différence entre ladite pluralité de points, ladite variation indiquant ladite au moins une discontinuité dans ladite structure physique.

2. Procédé selon la revendication 1, dans lequel l'amplitude dudit changement contrôlé de température varie sur ladite structure physique et ladite variation de l'amplitude est prise en compte dans la détection desdites irrégularités.

3. Procédé selon la revendication 1 ou 2, dans lequel ladite structure physique est faite de béton ou de béton conducteur.

4. Procédé selon la revendication 3, dans lequel ledit procédé est utilisé pour détecter et/ou localiser des nids de graviers dans le béton ou le béton conducteur et/ou pour analyser la qualité de la cimentation du béton ou du béton conducteur.

5. Procédé selon la revendication 1 ou 2, dans lequel ladite structure physique est une structure d'un réseau de chauffage urbain.

6. Procédé selon la revendication 1 ou 2, dans lequel ladite structure physique est faite de béton conducteur et ledit changement contrôlé de température est effectué au moyen des parties conductrices du béton.

7. Procédé selon la revendication 1 ou 2, dans lequel ladite structure physique est une construction en terre.

8. Procédé selon la revendication 1 ou 2, dans lequel ladite structure physique est un barrage en terre.

9. Procédé selon l'une quelconque des revendications 1 à 8, dans lequel ladite discontinuité est l'une des suivantes : un trou, une zone à gros grains, un nid de cailloux, une inclusion d'air, une fissure, un éclat, un nid de graviers, une zone congelée, de l'humidité, une fuite, une courbure ou une dislocation dans la structure physique, un objet étranger à l'intérieur de la structure, ou un fluide en mouvement à l'intérieur ou sur la surface de la structure physique.

10. Procédé selon l'une quelconque des revendications 1 à 9, dans lequel ledit procédé est utilisé pour surveiller l'état de la structure physique après que la construction de ladite structure physique a été terminée.

11. Procédé selon l'une quelconque des revendications 1 à 10, dans lequel ledit procédé est utilisé pour surveiller l'état de la structure physique pendant l'utilisation normale de ladite structure physique.

12. Procédé selon l'une quelconque des revendications 1 à 11, dans lequel l'amplitude dudit changement contrôlé de température est prédéterminée.

13. Procédé selon l'une quelconque des revendications 1 à 12, dans lequel l'amplitude dudit changement contrôlé de température est indépendante des résultats de mesure de température.

14. Agencement pour surveiller l'état d'une structure physique sensiblement homogène (20), comprenant :
- des moyens de mesure de température (27) comprenant un capteur à fibres optiques (21, 30, 32, 34) pour mesurer la température de ladite structure physique pour obtenir un ensemble de températures mesurées en une pluralité de points de ladite structure physique,
- un câble thermique (22, 31, 33a, 33b, 35) assemblé à proximité de ladite fibre optique (21, 30, 32, 34) pour provoquer un changement contrôlé de température dans ladite structure physique,
- ledit câble thermique (22, 31, 33a, 33b, 35) étant couplé à une unité de régulation de la température (26) qui est adaptée pour envoyer une impulsion thermique dans le câble thermique (22, 31, 33a, 33b, 35) pour provoquer un changement de température dont l'amplitude est uniforme sur ladite structure physique,
- des moyens (28) pour détecter des irrégularités dans lesdites températures mesurées, lesdites irrégularités indiquant au moins une discontinuité dans ladite structure physique,
- lesdits moyens (28) pour détecter lesdites irrégularités étant **caractérisés en ce qu'**ils sont adaptés pour :
- obtenir, pour ladite pluralité de points, un premier ensemble de températures mesurées avant ledit changement contrôlé de température et un second ensemble de températures mesurées après ledit changement contrôlé de température,
- calculer, pour ladite pluralité de points, une différence entre ledit premier ensemble de températures mesurées et ledit second ensemble de températures mesurées, et
- détecter une variation dans ladite différence entre ladite pluralité de points, ladite variation indiquant ladite au moins une discontinuité dans ladite structure physique.
